# EUROPEAN PATENT APPLICATION

(11) **EP 4 585 238 A1**
(43) Date of publication of application: **16.07.2025**
(21) Application number: 25150802.4
(22) Date of filing: 08.01.2025
(51) Int. Cl.: A61M 1/36, A61B 5/00

(54) **TUBING WITH INTEGRATED PRESSURE SENSOR**

(30) Priority: 10.01.2024 CN 202410037666
(71) Applicant: ChinaBridge (Shenzen) Medical Technology Co., Ltd., Shenzhen, Guangdong 518102 (CN)
(72) Inventor: LI, Yijiang, Shenzhen Guangdong, 518102 (CN)
(74) Representative: Pfenning, Meinig & Partner mbB

(57) **Abstract**

A fixing device for a pressure sensor arranged on a flexible tubing includes a housing and a fixing member. The fixing member is used to fix a sensor body. The fixing member is provided with a first cavity to accommodate the sensor body. The sensor is arranged in the first cavity. A protrusion is provided on the periphery of the first cavity. A second cavity is provided inside the housing at a position corresponding to the protrusion. The protrusion extends into the second cavity and is not connected with the second cavity. The housing and the fixing member do not contact each other. When the flexible tubing deforms or a relative displacement occurs between the flexible tubing and the fixing device, the connection between the pressure sensor and the flexible tubing maintains good sealing, which greatly reduces the risk of leakage at the connection between the pressure sensor and the flexible tubing.

## Description

### TECHNICAL FIELD

The present disclosure relates to the field of medical devices. More specifically, the present disclosure relates to a fixation device for a pressure sensor disposed on a flexible tubing.

### BACKGROUND

During cardiopulmonary bypass (CPB), monitoring real-time blood pressure is critical. Especially during extracorporeal membrane oxygenation (ECMO) operation, pressure monitoring of key components is necessary. Due to the inherent high blood flow and high flow rate during ECMO, safety requirements for the entire medical circulation tubing are very high. The principle of existing pressure sensors requires that a sensor probe be directly in contact with a liquid level to be detected, and also in contact with air to work properly. This requires drilling a hole at a certain position in the tubing, inserting the sensor probe into the tubing, and using a fixture to fix the pressure sensor on the tubing. Commonly used medical circulation tubes are mostly flexible tubes made of PU or PVC. If the flexible tubing is moved, blood penetration is possible from the hole where the pressure sensor is located. Blood penetration from the hole is relatively serious, so the sealing and fixation between the pressure sensor and the tubing is a problem that must be solved. This is also a need for risk management of medical device products.

During use, factors such as the orientation adjustment of the tubing itself, the doctor's observation of the tubing moving, and vibration during transportation may cause the flexible tubing to deform or cause relative displacement between the flexible tubing and the fixture, causing the probe to be in contact with the flexible tubing. This can lead to a sealing problem at the tubing joint. In the existing technology, such as the CN213748567U patent, the pressure sensor is relatively fixed to the housing when it is fixedly installed. When the flexible tubing is deformed or the flexible tubing and the fixed device are relatively displaced, the interaction force between the two can easily cause the sensor to move. This causes the needle detaches and leaks, causing the pressure sensor to not work properly.

### SUMMARY

In order to solve or alleviate the problems existing in the installation of pressure sensors in flexible tubes or tubing in the medical field, especially to reduce the risk of leakage at the installation location of pressure sensors in medical flexible tubing, this application provides a pressure sensor installed on the flexible tubing.

A housing and a fixing member are both set on a periphery of the flexible tubing and do not contact each other. The housing is set on the periphery of the fixing member at intervals. The fixing member is used to fix a sensor body. A probe is connected to the sensor body and penetrates the flexible tubing and is connected to the flexible tubing. The probe contacts liquid, such as blood, inside the flexible tubing.

The fixing member is provided with a first cavity for accommodating the sensor body, and the sensor body is arranged in the first cavity.

A protrusion is provided on the periphery of the first cavity, and a channel connected to the first cavity is provided inside the protrusion. A through hole is provided on the housing at a position corresponding to the protrusion. The through hole is connected with the protrusion for connecting the first cavity to the outside atmosphere.

A second cavity is provided inside the housing at a position corresponding to the protrusion. The protrusion extends into the second cavity and does not contact an inner wall of the second cavity, so that the protrusion can move inside the second cavity without being restricted by the housing.

Further, a limiting groove is provided at a nozzle of the protrusion close to the housing and/or at the passage opening of the protrusion away from the housing. A waterproof and breathable membrane is provided inside the limiting groove.

Furthermore, the length of the fixing member exceeds 2 times the length of the sensor body.

Furthermore, a side of the fixing member close to the flexible tubing is provided with an arc-shaped surface that fits the outer surface of the flexible tubing. The cross-sectional arc length of the arc-shaped surface is not less than 1/4 of the circumference of the cross-section of the flexible tubing.

Furthermore, the cross-sectional arc length of the arc-shaped surface is equal to 1/2 circumference of the cross-section of the flexible tubing.

Furthermore, a guide groove is provided on a surface where the fixing member is in contact with the flexible tubing.

Furthermore, an anti-slip member is provided between a side of the flexible tubing away from the fixing member and the housing. The anti-slip member is used to prevent axial movement of the flexible tubing and the housing.

Furthermore, the sensor body is flat.

Further, the side of the sensor body close to the flexible tubing is attached to the flexible tubing.

Furthermore, the probe is arranged vertically on the sensor body.

Furthermore, both ends of the housing are respectively fixed on the outer surface of the flexible tubing through fastening devices.

Furthermore, the fastening devices include a threaded ring and a nut, the flexible tubing penetrates the threaded ring and the nut, and the nut is threadedly connected to the threaded ring.

In an embodiment of the present application, a second cavity is provided inside the housing at a position corresponding to the protrusion. The protrusion extends into the second cavity, and the protrusion does not contact the inner wall of the second cavity. In this arrangement, the protrusion is in the second cavity. The internal movement of the protrusion is not restricted by the housing. When the flexible tubing deforms or the relative displacement occurs between the flexible tubing and the fixing member, the protrusion can move inside the second cavity while ensuring normal operation of the pressure sensor. There will be no interaction force between the two, which will not cause the fixing member, sensor body and probe to separate from the flexible tubing. Thus, ensuring the sealing of the device when the flexible tubing deforms, or relative displacement occurs between the flexible tubing and the fixing member. This arrangement prevents leakage of blood from the tubing.

### BRIEF DESCRIPTION OF THE DRAWINGS

The drawings described here are used to provide a further understanding of the present application and constitute a part of the present application. The illustrative embodiments of the present application and their descriptions are used to explain the present application and do not constitute an improper limitation of the present application. Some specific embodiments of the present application will be described in detail below by way of illustration and not limitation with reference to the accompanying drawings. In the drawings, like reference numerals designate the same or similar components or portions. It will be understood by those skilled in the art that these drawings are not necessarily drawn to scale. In the drawings:
Figure 1 is a three-dimensional cross-sectional view of a fixation device according to the embodiment of the present application;
Figure 2 is a cross-sectional front view of the fixing device according to the embodiment of the present application;
Figure 3 is a three-dimensional structural diagram of a fixing member according to the embodiment of the present application;
Figure 4 is a bottom view of the fixing member according to the embodiment of the present application;
Figure 5 is an enlarged view of position A in Figure 2 according to the embodiment of the present application;
Figure 6 is a side view of the fixing member according to the embodiment of the present application;
Figure 7 is a cross-sectional front view of the housing according to the embodiment of the present application;
Figure 8 is a schematic diagram of the three-dimensional structure of the housing according to the embodiment of the present application;
Figure 9 is an enlarged view of B in Figure 2 according to the embodiment of the present application.

In the Figures: 1. Housing; 11. Through hole; 12. Second cavity; 13. Mounting hole; 14. Glue injection hole; 2. Fixing member; 21. First cavity; 22. Protrusion; 23. Channel; 24. Limit groove; 25. Escape opening; 26. Reinforcement rib; 27. Platform; 28. Diversion groove; 3. Flexible tubing; 4. Sensor body; 41. Probe; 5. Waterproof and breathable membrane; 6 , Anti-slip member; 7. Fastening device; 71. Threaded ring; 72. Nut; 8. Connector.

While the disclosure is amenable to various modifications and alternative forms, specific embodiments have been shown by way of example in the drawings and are described in detail below. The intention, however, is not to limit the disclosure to the particular embodiments described. On the contrary, the disclosure is intended to cover all modifications, equivalents, and alternatives falling within the scope of the disclosure as defined by the appended claims.

### DETAILED DESCRIPTION

In order to enable those in the technical field to better understand the solution of the present application, the technical solution in the embodiment of the present application will be clearly and completely described below in conjunction with the drawings in the embodiment of the present application. The described embodiments are only part of the embodiments of the present application, rather than all the embodiments. Based on the embodiments in this application, all other embodiments obtained by those of ordinary skill in the art without creative efforts should fall within the scope of protection of this application.

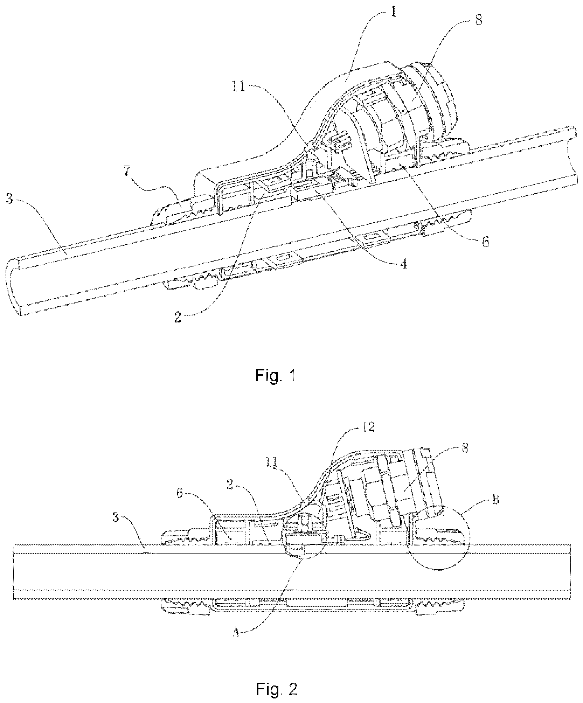

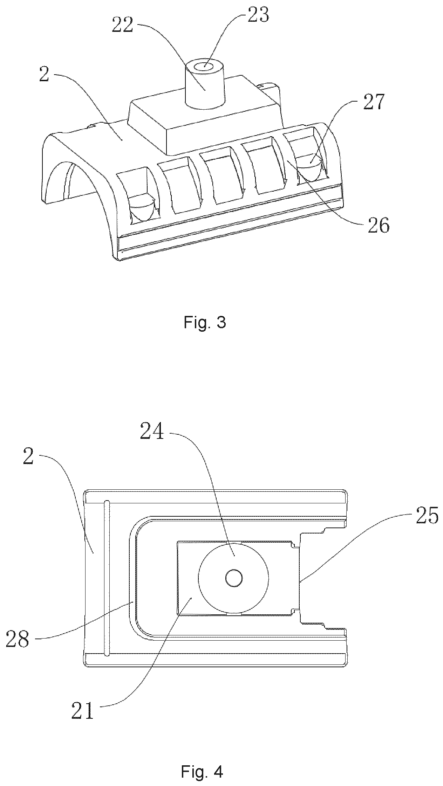

As shown in Figures 1-5, the embodiment of the present application provides a fixing device for a pressure sensor arranged on a flexible tubing, including a housing 1 and a fixing member 2. The pressure sensor includes a sensor body 4 and a probe 41.

The housing 1 and the fixing member 2 are both set on a periphery of the flexible tubing 3 and do not contact each other. The housing 1 is set on the periphery of the fixing member 2 at intervals. The fixing member 2 is used to fix the sensor body 4 and is connected to the sensor body 4. The probe 41 penetrates the flexible tubing 3 and comes into contact with the liquid inside the flexible tubing 3, such as blood. It is necessary to punch a hole in the flexible tubing 3 that matches the size of the probe 41, and then insert the probe 41 into the hole, so that the probe 41 is in contact with the liquid inside the flexible tubing 3. Any gap between the sensor body 4 and the flexible tubing 3 is filled with UV glue, and then irradiated and cured by an ultraviolet UV lamp.

The fixing member 2 is provided with a first cavity 21 that accommodates the sensor body 4. The sensor body 4 is set in the first cavity 21. At this time, it is necessary to apply UV glue on the side of the fixing member 2 and the flexible tubing 3 to make it fit. The fixing member 2 is fixedly installed on the flexible tubing 3.

A protrusion 22 is provided on the periphery of the first cavity 21, and a channel 23 connected with the first cavity 21 is provided inside the protrusion 22. A through hole 11 is provided on the housing 1 at a position corresponding to the protrusion 22. The through hole 11 in communication with the protrusion 22 is used to connect the first cavity 21 to the outside atmosphere.

A second cavity 12 is provided inside the housing 1 at a position corresponding to the protrusion 22. The protrusion 22 extends into the second cavity 12 and does not contact the inner wall of the second cavity 12, so that the protrusion 22 is in the second cavity 12. Movement of the protrusion 22 within the second cavity 12 is not restricted by the housing 1.

In this embodiment, the sensor body 4 is installed on the flexible tubing 3 by the fixing member 2. Space between the sensor body 4, the fixing member 2, and the flexible tubing 3 is filled and fixed by applying UV glue, which improves the connection between the pressure sensor and the flexible tubing 3 and increases the tightness of the joint.

By arranging the protrusion 22 on the fixing member 2, the protrusion 22 allows the sensor body 4 to be connected to the outside atmosphere, so that the sensor body 4 can work normally. The second cavity 12 is provided inside the housing 1 at a position corresponding to the protrusion 22. The protrusion 22 extends into the second cavity 12. The protrusion 22 does not contact the inner wall of the second cavity 12. In this configuration, the protrusion 22 moves inside the second cavity 12 without being restricted by the housing 1. When deformation the flexible tubing 3 occurs or relative displacement occurs between the flexible tubing 3 and a fastening device 7, the protrusion 22 can move inside the second cavity 12 ensuring normal operation of the sensor body 4. When the flexible tubing 3 is deformed or relative displacement occurs between the flexible tubing 3 and the fastening device 7, any generated Interaction force will not cause the fixing member 2, the sensor body 4, and the probe 41 to separate from the flexible tubing 3. Thereby ensuring that the device remains sealed and leakage is prevented.

In one embodiment, a limit groove 24 is provided near the mouth of the protrusion 22 of the housing 1 and/or at the mouth of the channel 23 away from the protrusion 22 of the housing 1. A waterproof and breathable membrane 5 is provided inside the limit groove 24.

In this embodiment, by disposing the waterproof and breathable membrane 5 at the protrusion 22 close to the mouth of the housing 1, external moisture, dust, etc. is prevented from entering the inside of the first cavity 21. Moisture, dust, etc. can damage the sensor body 4 and prevent it from working normally. At the same time, the waterproof and breathable membrane 5 allows exposure to atmosphere, so that the sensor body 4 can work normally.

In one embodiment, a plurality of reinforcement ribs 26 are arranged in an array on both sides of the fixing member 2. In this embodiment, by arranging a plurality of reinforcing ribs 26 on both sides of the fixing member 2, the strength of the fixing member 2 is improved.

In one embodiment, several platforms 27 are provided on the fixing member 2. In this embodiment, the platforms 27 are provided on the fixing member 2 to facilitate the installation and disassembly of the fixing member 2.

In one embodiment, a guide groove 28 is provided on a surface where the fixing member 2 is in contact with the flexible tubing 3. In this embodiment, the guide groove 28 is provided on the surface where the fixing member 2 and the flexible tubing 3 fit together. The guide groove 28 provides a guide for injecting glue. The injected glue passes through the guide groove 28 to evenly distribute the injected glue, so that the fixing member 2 can be better fixed on the flexible tubing 3.

In addition, the guide groove 28 can be used as a basis for judging whether the fixing member 2 and the flexible tubing 3 is sufficiently bonded or not. If there are no bubbles in the guide groove 28, airtight performance of the bonding between the fixing member 2 and the flexible tubing 3 can be guaranteed. Preferably, the guide groove 28 is a U-shaped groove.

In one embodiment, the length of the fixing member 2 exceeds 2 times the length of the sensor body 4. In this embodiment, the length of the fixing member 2 is set to exceed twice the length of the sensor body 4 in order to provide a larger contact area and more fixing points, thus increasing the stability of the connection. This reduces looseness and ensures that the fixing member 2 remains firmly in place during shaking. Longer fixing members 2 usually have higher strength and rigidity and can provide greater strength reserves, allowing the fixing member 2 to withstand greater forces and loads. A longer fixing member 2 can disperse force and stress and reduce the concentration of local stress, thereby reducing the risk of fatigue and damage of the fixing member 2 and extending the service life of the fixing member 2. Therefore, reducing the frequency of repairs and replacements.

In some embodiments, the length of the sensor body 4 is 8-10 mm. Preferably, the length of the sensor body 4 is 9.18 mm. In some embodiments, the length of the fixing member 2 is 20-30 mm. Preferably, the length of the fixing member 2 is 24.44 mm.

In one embodiment, the length of the housing 1 exceeds 2 times the length of the fixing member 2. In this arrangement, the flexible tubing 3 at both ends of the fixing member 2 can always be kept on the same straight line, so that the part of the flexible tubing 3 located inside the housing 1 is not prone to bending and deformation. Specifically, the length of the housing 1 is 70-90mm. Preferably, the length of the housing 1 is 78.95mm.

In one embodiment, the sensor body 4 is flat. In this embodiment, the flat sensor body 4 is provided with a thin design, making the sensor body 4 compact and more suitable for application scenarios with limited space. The flat sensor body 4 can be easily installed in a small space or on a curved surface. Due to the compact structure of the body 4, the contact area between the sensing element and the measured medium is large, and the response speed is relatively fast. The flat sensor body 4 has a fast response and small inertia, making it more suitable for dynamic pressure measurement.

In one embodiment, the side of the sensor body 4 close to the flexible tubing 3 is attached to the flexible tubing 3. In this embodiment, by designing the side of the sensor body 4 close to the flexible tubing 3 to fit into the flexible tubing 3, the gap between the sensor body 4 and the flexible tubing 3 can be reduced, thereby improving the flexibility of the sensor body 4 and the flexible tubing 3.

In one embodiment, the probe 41 is arranged vertically on the sensor body 4. In this embodiment, by arranging the probe 41 perpendicular to the sensor body 4, the influence of a fluctuation of the liquid in the tube on the measurement of the sensor body 4 can be reduced. The vertical arrangement can maximize the vertical component of the pressure of the medium acting on the probe 41. The horizontal component of the fluctuation is relatively small, thereby improving the stability and accuracy of the measurement. The vertical arrangement can maximize the contact area between the sensor probe 41 and the measured medium, thereby improving the sensitivity of the sensor. The vertical arrangement reduces errors and drifts due to gravity or other factors.

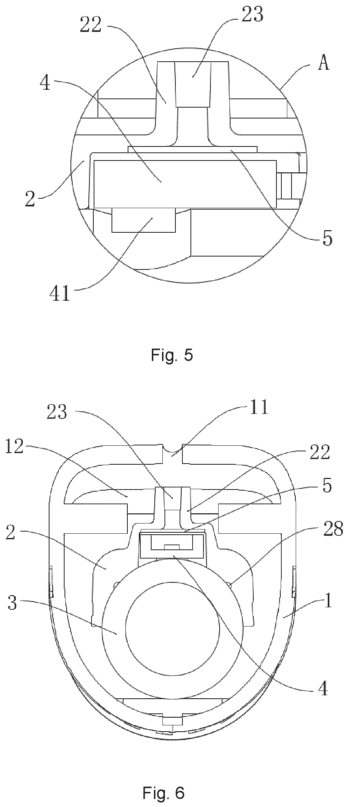

As shown in Figure 6, in one embodiment, the side of the fixing member 2 close to the flexible tubing 3 is provided with an arc surface that fits the outer surface of the flexible tubing 3. The cross-sectional arc length of the arc surface is not less than 1/4 circumference cross section of the flexible tubing 3.

In this embodiment, setting the arc length of the arc-shaped surface to be no less than 1/4 of the circumference of the cross-section of the flexible tubing 3 increases the contact area between the fixing member 2 and the flexible tubing 3. This increases the friction between the surface of the fixing member 2 and the flexible tubing 3, which increases the anti-sliding ability of the fixing member 2, and reduces the possibility of loosening and shaking. The larger contact area can disperse a load and reduce the concentration of local stress, which can reduce the stress concentration on the surface of the fixing member 2 and the flexible tubing 3. Further reducing the risk of deformation and damage of the fixing member 2. A larger contact area increases friction between the fixing member 2 and the surface of the flexible tubing 3 making it more resistant to impact caused by vibration, which can improve the anti-seismic performance of the fixing member 2. In addition, the larger the contact area between the fixing member 2 and the flexible tubing 3, the more difficult it is for the fixing member 2 to break away from the surface of the flexible tubing 3.

Preferably, the arc length of the arc-shaped surface is equal to 1/2 the circumference of the cross-section of the flexible tubing 3.

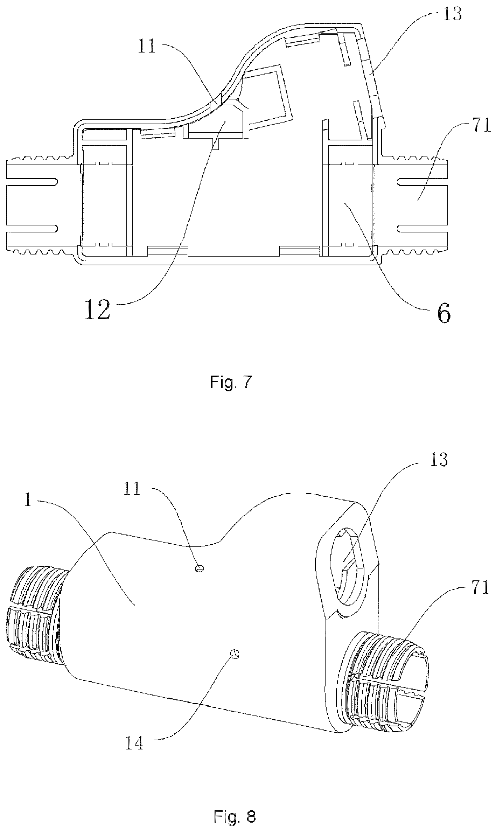

As shown in Figures 7-8, in one embodiment, an anti-slip member 6 is provided between the side of the flexible tubing 3 away from the fixing member 2 and the housing 1. The anti-slip member 6 is used to prevent the flexible tubing 3 from colliding with the housing 1.

In this embodiment, by disposing an anti-slip member 6 between the side away from the flexible tubing 3 and the housing 1, the anti-slip member 6 can prevent the flexible tubing 3 from axially moving inside the housing 1 under the action of external force.

In one embodiment, the housing 1 is provided with a mounting hole 13, and the mounting hole 13 is provided with a connector 8. The fixing member 2 is provided with an escape opening 25, and the escape opening 25 is connected to the first cavity 21. The sensor body 4 extends from the escape opening 25 and is electrically connected to the connector 8.

In this embodiment, the connector 8 is installed at the mounting hole 13 on the housing 1. The fixing member 2 is provided with an escape opening 25, and one end of the sensor body 4 extends from the escape opening 25. Then the sensor body 4 is electrically connected to the connector 8, and the connector 8 is also connected to an external host.

In one embodiment, the housing 1 is provided with a glue injection hole 14. In this embodiment, after the installation of the housing 1 is completed, silicone can be injected into the interior of the housing 1 through the glue injection hole 14 until it overflows. The purpose is to seal the housing 1. The internal space within the housing 1 is filled so that the internal components are relatively fixed, improving the stability and sealing of the device.

Specifically, the filling amount is 5-8g. Before injecting glue, pins will be used to block the channel 23 and through hole 11 of the protrusion 22 in order to ensure that the channel 23 and through hole 11 can be avoided during the glue injection and curing process. Following injection of glue into through hole 11, and solidification of the glue, the pins can be removed to ensure that the sensor chip can be smoothly connected to the atmosphere for calibration. In addition, the channel 23 is provided with a platform set at the bottom to prevent the pins from breaking the waterproof and breathable membrane 5. The channel 23 is provided with a draft angle, that is, a demolding angle, which is set in the mold cavity to facilitate mold removal. The orientation of the draft angle depends on the internal and external dimensions of the plastic part. In order to allow the molded product to be ejected smoothly from the mold, the draft angle must be set on the wall in the same direction as the opening and closing of the mold (including the side core and reinforcing ribs) to facilitate demolding.

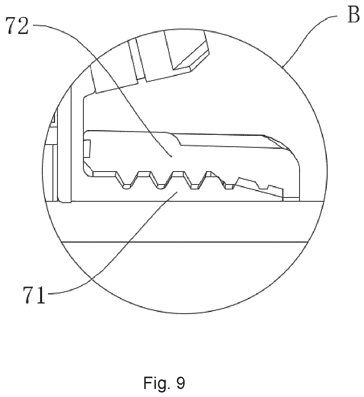

As shown in FIG. 9, in one embodiment, both ends of the housing 1 are respectively fixed on the outer surface of the flexible tubing 3 through fastening devices 7. In this embodiment, fastening devices 7 are provided at both ends of the housing 1 to prevent the housing 1 from sliding on the flexible tubing 3

In one embodiment, a fastening device 7 includes a threaded ring 71 and a nut 72. The flexible tubing 3 passes through the threaded ring 71 and the nut 72, and the nut 72 is threadedly connected to the threaded ring 71. In this embodiment, the nut 72 is screwed on the threaded ring 71 so that the nut 72 is threadedly connected to the threaded ring 71, thereby fixing the housing 1 on the flexible tubing 3.

In one embodiment, several notches are provided on the threaded ring 71, and the inner diameter of the end of the nut 72 close to the housing 1 is larger than the inner diameter of the end of the nut 72 farther away from the housing 1.

In this embodiment, several notches are provided on the threaded ring 71 and the inner diameter of the end of the nut 72 close to the housing 1 that is larger than the inner diameter of the end far away from the housing 1. When the nut 72 is gradually twisted on the threaded ring 71, the inner diameter of the end of the nut 72 away from the housing 1 becomes smaller and smaller, and the gap in the threaded ring 71 will gradually become smaller. This causes the opening of the threaded ring 71 to gradually shrink, thereby achieving the purpose of tightening the flexible tubing 3. The clamping effect prevents the housing 1 from sliding on the flexible tubing 3, further improving the stability of the connection between the housing 1 and the flexible tubing 3.

In one embodiment, the housing 1 includes a first housing and a second housing, and the first housing and the second housing are detachably connected. In this embodiment, the housing 1 is assembled by the first housing and the second housing. The purpose of this arrangement is to facilitate installation, so that the housing 1 can be quickly installed on the flexible tubing 3. Preferably, the after the first housing and the second housing are joined together, the connection between the first housing and the second housing can be further strengthened through the fastening device 7.

In one embodiment, the fixture installation steps are as follows:
Drill a hole in the flexible tubing 3 and extend the probe 41 into the flexible tubing 3. Install the sensor body 4 on the outer surface of the tubing and apply UV glue at the connection gap between the sensor body 4 and the flexible tubing 3. Cure the UV glue by lamp irradiation. Electrically connect the connector to the sensor body 4. Apply UV glue on the arc-shaped surface where the fixing member 2 and the flexible tubing 3 fit. Place the waterproof and breathable membrane 5 in the limiting groove 24. Locate the sensor body 4 in the first cavity 21. Install the fixing member 2 to the tubing, and then inject glue into the guide channel 28. Install the connector to the mounting hole 13 of the housing 1. Align the protrusion 22 on the fixing member 2 and put it into the second cavity 12. Set the anti-slip member 6 on the flexible tubing 3, and then install the housing 1 on the tubing. Thread the nut 72 on the thread ring 71. Block the channel 23 of the protrusion 22 and the through hole 11 and inject silicone into the interior of the housing 1 through the glue injection hole 14.

Finally, it should be noted that the above embodiments are only used to illustrate the technical solution of the present application, but not to limit it; although the present application has been described in detail with reference to the foregoing embodiments, those of ordinary skill in the art should understand that the technical solutions described in the foregoing embodiments can still be modified, or some or all of the technical features can be equivalently replaced, and these modifications or substitutions do not deviate from the essence of the corresponding technical solutions from the technical solutions of the embodiments of the present application.

## Claims

1. A fixing device for a pressure sensor arranged on a flexible tubing, the fixing device comprising:
a housing and a fixing member;
the housing and the fixing member are both set on a periphery of the flexible tubing and do not contact each other, the housing is set on a periphery of the fixing member at intervals, the fixing member is used to fix a flat sensor body and is connected to the sensor, a probe connected to the sensor body penetrates the flexible tubing and contacts a liquid inside the flexible tubing;
the fixing member is provided with a first cavity that accommodates the sensor body, and the sensor body is disposed in the first cavity;
a protrusion is provided on a periphery of the first cavity, a passage communicating with the first cavity is provided inside the protrusion, and a through hole is provided on the housing at a position corresponding to the protrusion to connect the first cavity to an outside atmosphere; and
a second cavity is provided inside the housing at a position corresponding to the protrusion, and the protrusion extends into the second cavity and does not contact an inner wall of the second cavity.

2. The fixing device for a pressure sensor installed on a flexible tubing according to claim 1, wherein a length of the fixing member exceeds twice a length of the sensor body.

3. The fixing device for a pressure sensor arranged on a flexible tubing according to claim 1, wherein the fixing member is provided with an arc-shaped surface on one side close to the flexible tubing that fits the outer surface of the flexible tubing, and a cross-sectional arc length of the arc-shaped surface is not less than 1/4 of a circumference of a cross-section of the flexible tubing.

4. The fixing device for a pressure sensor arranged on a flexible tubing according to claim 3, **characterized in that** the cross-sectional arc length of the arc-shaped surface is equal to 1/2 of the circumference of the cross-section of the flexible tubing.

5. The fixing device for a pressure sensor arranged on a flexible tubing according to claim 1, **characterized in that** a guide groove is provided on a surface of the fixing member that is in contact with the flexible tubing.

6. The fixing device for a pressure sensor arranged on a flexible tubing according to claim 1, **characterized in that** an anti-slip member is provided between a side of the flexible tubing away from the fixing member and the housing, and the anti-slip member prevents axial movement between the flexible tubing and the housing.

7. The fixing device for a pressure sensor arranged on a flexible tubing according to claim 1, wherein a side of the sensor body close to the flexible tubing is in contact with the flexible tubing.

8. The fixing device for a pressure sensor arranged on a flexible tubing according to claim 7, wherein the probe is arranged vertically on the sensor body.

9. The fixing device for a pressure sensor installed on a flexible tubing according to claim 1, wherein two ends of the housing are respectively fixed on an outer surface of the flexible tubing with fastening devices.

10. The fixing device for a pressure sensor arranged on a flexible tubing according to claim 9, wherein the fastening device includes a threaded ring and a nut threadedly connected to the threaded ring, and the flexible tubing runs through the threaded ring and the nut.

11. A fixing device for a pressure sensor arranged on a flexible tubing, the fixing device comprising:
a fixing member configured for securing a sensory body, the fixing member including:
a first cavity that accommodates the sensor body; and
a protrusion provided on a periphery of the first cavity; and
a housing surrounding the fixing member, the housing including:
a second cavity at a position corresponding to the protrusion, wherein the protrusion extends into the second cavity.

12. The fixing device for a pressure sensor installed on a flexible tubing according to claim 11, wherein the sensor body includes a probe, and the probe contacts a liquid inside the flexible tubing.

13. The fixing device for a pressure sensor installed on a flexible tubing according to claim 11, wherein a length of the fixing member exceeds twice a length of the sensor body.

14. The fixing device for a pressure sensor arranged on a flexible tubing according to claim 11, wherein the fixing member is provided with an arc-shaped surface on one side close to the flexible tubing that contacts the outer surface of the flexible tubing.

15. The fixing device of claim 14, wherein a cross-sectional arc length of the arc-shaped surface is not less than 1/4 of a circumference of a cross-section of the flexible tubing.

16. The fixing device for a pressure sensor arranged on a flexible tubing according to claim 11, wherein the fixing member further includes a guide groove on a surface that is in contact with the flexible tubing.

17. The fixing device for a pressure sensor arranged on a flexible tubing according to claim 11, further comprising:
an anti-slip member configured to prevent axial movement between the flexible tubing and the housing.

18. The fixing device for a pressure sensor arranged on a flexible tubing according to claim 11, wherein the fixing member includes a plurality of reinforcement ribs arranged in an array.

19. The fixing device for a pressure sensor arranged on a flexible tubing according to claim 11, wherein the fixing member includes a limit groove near a mouth of the protrusion.

20. The fixing device for a pressure sensor installed on a flexible tubing according to claim 19, wherein a waterproof and breathable membrane provided inside the limit groove.
